# EUROPÄISCHE PATENTSCHRIFT

(11) **EP 0 348 581 B1**
(45) Veröffentlichungstag und Bekanntmachung des Hinweises auf die Patenterteilung: **24.02.1993**
(21) Anmeldenummer: 88810599.6
(22) Anmeldetag: 01.09.1988
(51) Int. Cl.: A61F 5/02, A61B 17/58

(54) **Implantat zur Fixierung von Wirbeln**
Vertebrae fixation implant
Implant pour la fixation de vertèbres

(30) Priorität: 10.06.1988 CH 2233/88
(43) Veröffentlichungstag der Anmeldung: 03.01.1990
(73) Patentinhaber: SYNTHES AG, CH-7002 Chur (CH)
(72) Erfinder: Aebi, Max, Dr., CH-3011 Bern (CH); Mathys, Robert, Jr., CH-2544 Bettlach (CH)
(74) Vertreter: Eder, Carl E.

(56) Entgegenhaltungen:
- CH-A- 639 264
- DE-A- 2 843 711
- DE-A- 3 132 520
- DE-A- 3 515 678
- DE-U- 8 703 022
- FR-A- 2 405 063
- FR-A- 2 557 933
- GB-A- 2 178 323

## Beschreibung

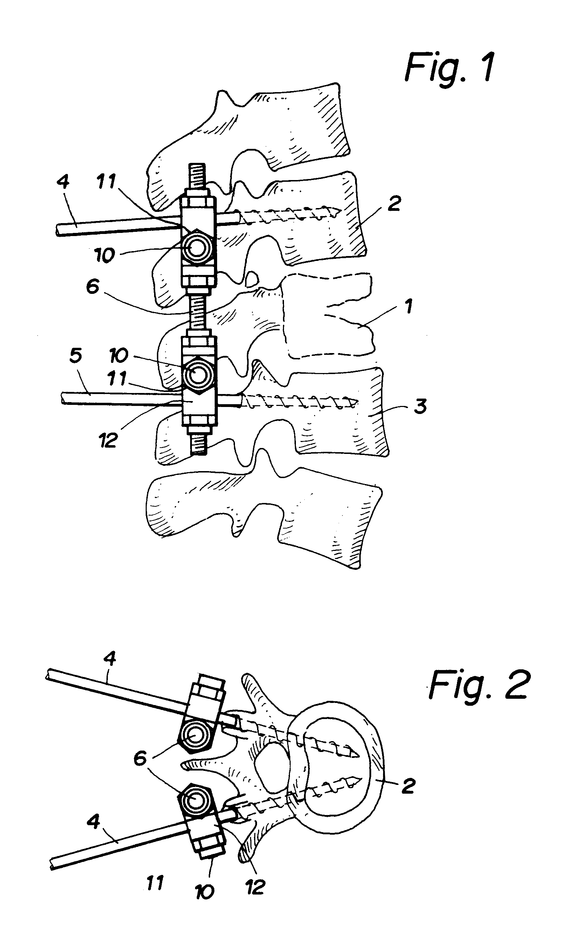

Die vorliegende Erfindung betrifft einen zum steifen Verbinden eines Wirbels der Wirbelsäule mit mindestens einem zweiten Wirbel einer Wirbelsäule dienenden Fixateur intern nach dem Oberbegriff des Patentanspruchs. Derartige Fixateure sind in der Fachwelt bekannt und beispielsweise im Bulletin Synthes der Arbeitsgemeinschaft für Osteosynthesefragen Nr. 70 vom März 1986 beschrieben. Die Figuren 1 und 2 der beiliegenden Zeichnung sind dieser Publikationen entnommen.

In der Zeichnung zeigt
die Figur 1 eine Seitenansicht von fünf Wirbeln, von denen der mittlere defekt ist und die beiden benachbarten durch einen Fixateur intern miteinander verbunden sind, während in
der Figur 2 eine Draufsicht auf einen Wirbel mit zwei eingesetzten Schanzschen Schrauben dargestellt ist;
die Figur 3 zeigt einen Ausschnitt aus einer Gewindestange mit einem auf ihr sitzenden erfindungsgemässen Verbindungselement,
die Figur 4 einen Längsschnitt durch die Klemmvorrichtung dieses Verbindungselements mit eingesetzer Schanzscher Schraube, geschnitten senkrecht zur Hülse für die Gewindestange, also gemäss der Linien IV - IV der Figur 3, und
die Figur 5 einen Längsschnitt nach der Linie V - V der Figur 3, ohne die Gewindestange.

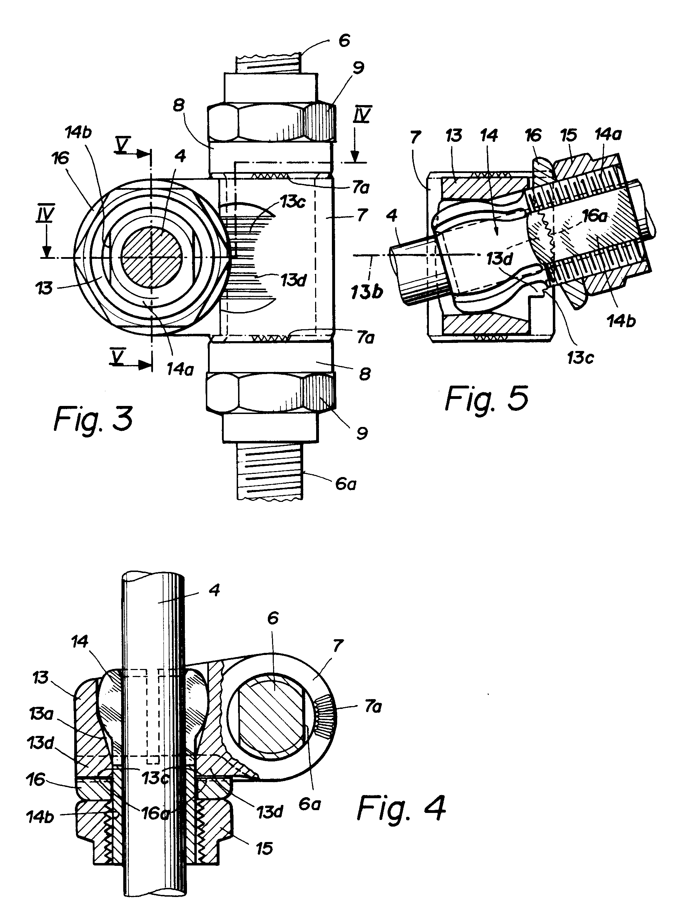

Wie man den Figuren 1 und 2 entnehmen kann, ist es bekannt, zum starren Verbinden der beiden zu einem verletzten Wirbel 1 benachbarten Wirbel 2 und 3 in jeden der beiden Wirbel 2 und 3 je zwei Schanzsche Schrauben 4 bzw. 5 einzusetzen, wobei je eine Schanzsche Schraube 4 des oberen Wirbels 2 über einen Gewindestab 6 mit der entsprechenden Schanzschen Schraube 5 des unteren Wirbels 3 starr verbunden ist. Die starre Verbindung zwischen einer Schanzschen Schraube und dem Gewindestab 6 wird jeweils durch ein Verbindungselement gewährleistet. Jedes dieser Verbindungselemente weist eine auf dem Gewindestab 6 verschiebbare und verdrehbare Hülse auf, wie das auch beim erfindungsgemässen Verbindungselement der Fall ist, wo diese Hülse in den Figuren 3 bis 5 mit 7 bezeichnet ist. Diese Hülse hat auf jeder Seite eine gezahnte Stirnfläche 7a. Mit jeder dieser Zahnungen kämmt die Zahnung je einer Unterlagscheibe 8, die auf dem Gewindestab 6 sitzt und durch eine Mutter 9 gegen die Hülse gepresst wird. Da der Gewindestab 6 mindestens eine Abflachung 6a aufweist und die Öffnung der Unterlagscheibe 8 mit der Querschnittsform des Gewindestabes 6 übereinstimmt, lässt sich so die Hülse 7 durch die beiden Muttern 9 an jedem Ort unverschiebbar und unverdrehbar fixieren. Bei dem bekannten, in der Figur 1 und 2 dargestellten Verbindungselement ist die Hülse mit einem senkrecht zur Hülsen-Achse angeordneten Gewindebolzen 10 versehen, auf welchem eine als Klemmvorrichtung für die Schanzsche Schraube dienende Klemmbacke 12 sitzt, die sich mittels einer Mutter 11 fest auf die Schanzsche Schraube 4 pressen lässt um diese Schanzsche Schraube fest mit der auf dem Gewindestab 6 fixierten Hülse 7 zu verbinden. Diese Art des Festklemmens der Schanzschen Schraube hat zwei Nachteile:

Einerseits ist die Mutter 11 mit einem Schraubenschlüssel nur sehr schwer zugänglich und andererseits ist die Spannkraft, die durch die Mutter auf die Klemmbacke übertragen werden kann, an sich nicht besonders gross und sie wird zudem noch dadurch verringert, dass sich die beim Anspannen der Klemmbacke einstellende elastische Verformung plastisch abbaut.

Diese beiden Nachteile weist nun die Klemmvorrichtung des erfindungsgemässen Fixateurs intern nicht auf.

Wie man insbesondere aus den Figuren 3 und 4 der Zeichnung ersehen kann, wird hier die Klemmvorrichtung gebildet durch ein seitlich an der Hülse 7 angeordnetes Auge 13 mit einer konischen Öffnung 13a, einer in dieser Öffnung 13a sitzenden, zum Festklemmen der Schanzsche Schraube 4 dienenden, am einen Ende mit einem Aussengewinde 14a versehenen Spannzange 14 und einer auf deren Aussengewinde 14a sitzenden Mutter 15, um die Spannzange 14 in die konische Öffnung 13a hineinzuziehen und so durch Verkleinerung der lichten Weite der Spannzange 14 die Schanzsche Schraube 4 unverrückbar festzuhalten. Damit sich nun beim Anziehen oder Lösen, d.h. beim Drehen der Mutter 15 die Spannzange 14 nicht dreht, ist das Aussengewinde 14a der Spannzange 14 mit mindestens einer, vorzugsweise mit zwei einander gegenüberstehenden Abflachungen 14b versehen, während die konische Öffnung 13a an ihrem engeren Ende eine dazu passende Verengung aufweist, wodurch das Verdrehen der Spannzange 14 verunmöglicht wird.

Da es aber nötig ist, dass die Schanzschen Schrauben 4 und 5 während der Operation gekippt und auch in einer Stellung fixiert werden können, in denen sie mit der Achse 13b der konischen Öffnung einen Winkel bilden, weist der durch das Hineinziehen in die konische Öffnung zusammendrückbare Abschnitt der Spannzange nicht den bei Spannzangen üblichen Aussen-Konus (wie er beispielsweise in der FR-A-2 405 063 dargestellt ist) sondern eine kugelförmige Aussenfläche auf, wie das aus den Figuren 4 und 5 ersichtlich ist. Damit nun beim Spannen der Spannzange, also beim Anziehen der Mutter 15 die gewählte Stellung beibehalten wird, ist zwischen dieser Mutter 15 und dem Ende der konischen Öffnung eine Unterlagsscheibe 16 angeordnet, die einen mit einer Zahnung 16a versehenen kreissegmentartigen Ausschnitt aufweist, wobei diese Zahnung 16a mit einer dazu passenden Zahnung 13c an den beiden Endlappen 13d am Ende der konischen Öffnung kämmt. Eine Spannhülse mit kugeliger Aussenfläche ist bereits zur Verwendung bei dem in der DE-35 15 678 A1 vorgeschlagenen Fixateur extern angegeben. Da dort jedoch die kugelige Aussenfläche in einer Spannvorrichtung mit kugeliger Innenfläche gelagert ist, die ihrerseits durch eine Mutter oder eine Spannbride gegen eine Kegelfläche gezogen wird, wird bei dieser vorveröffentlichten Ausführungsform einerseits ein zusätzlicher Bestandteil benötigt, andererseits fehlt jede Sicherung, die ein Verschwenken der Kugel beim Anziehen der Mutter bzw. der Spannbride verhindern könnte.

Durch die vorstehend beschriebene Konstruktion weist das Verbindungselement also die Eingangs geschilderten Nachteile der bekannten Verbindungselemente nicht mehr auf: Einerseits ist eine Klemmung mit einer Spannzange wesentlich wirkungsvoller als die Klemmung mit einer Klemmbacke und andererseits lässt sich mittels eines einmal eingesteckten Steckschlüssels die mit ihrer Achse in der Blickrichtung des Chirurgen liegenden Mutter zum Hineinziehen der Spannzange wesentlich einfach betätigen als eine nur von der Seite her sichtbare Mutter, für deren Betätigung ein Schraubenschlüssel benötigt wird, der pro Umdrehung etwa sechs mal frisch angesetzt werden muss.

## Patentansprüche

1. Zum steifen Verbinden eines Wirbels der Wirbelsäule mit mindestens einem zweiten Wirbel dieser Wirbelsäule dienender Fixateur intern bestehend aus zwei Schanzschen Schrauben (4, 5) pro Wirbel, zwei Gewindestäben (6) und pro Schanzsche Schraube einem Verbindungselement, um diese fest mit einem der Gewindestäbe zu verbinden, wobei jedes Verbindungselement eine auf dem Gewindestab (6) verschieb- und verdrehbare Hülse (7) aufweist mit Mitteln, um diese Hülse (7) gegen das Verschieben und Verdrehen zu sichern, sowie einer Klemmvorrichtung, um die Schanzsche Schraube festzuhalten, dadurch gekennzeichnet, dass diese Klemmvorrichtung gebildet wird durch ein seitlich an der Hülse (7) angeordnetes Auge (13) mit konischer Öffnung (13a), einer in dieser Öffnung sitzenden, mit einem Aussengewinde (14a) versehenen Spannzange (14) für die Schanzsche Schraube (4, 5) und einer auf dem Aussengewinde sitzenden Mutter (15), um die Spannzange (14) in die konische Öffnung (13a) hineinzuziehen und so die lichte Weite der Spannzange (14) zu verkleinern, um dadurch die Schanzsche Schraube festzuhalten, wobei das Aussengewinde (14a) der Spannzange mindestens eine Abflachung (14b) und der entsprechende Abschnitt der konischen Öffnung (13a) eine dazu passende Verengung aufweist, um das Verdrehen der Spannzange zu verhindern, dass der durch das Hineinziehen in die konische Öffnung zusammendrückbare Abschnitt der Spannzange eine kugelförmige Aussenfläche und die konische Öffnung zwei Verlängerungslappen (13d) aufweist, die ein Verschwenken der Spannzange (14) in einer zur Hülsenachse (7) parallelen Ebene ermöglichen, und dass die Verlängerungslappen (13d) mit einer auf einer zylindrisch gebogenen Fläche angebrachten Zahnung (13c) versehen sind, die mit der Zahnung (16a) kämmt, mit welcher eine auf das Aussengewinde (14a) der Spannzange (14) aufgeschobene und dort auch durch die Mutter (15) festgehaltene Unterlagscheibe (16) versehen ist.

## Claims

1. Implant which serves for the stiff connection of a vertebra of the spinal column with at least one second vertebra of this spinal column and which internally consists of two entrenching screws (4, 5) per vertebra, two threaded rods (6) and one connecting element per entrenching screw for fixedly connecting this with one of the threaded rods, wherein each connecting element has a sleeve (7) which is slidable and rotatable on the threaded rod (6) and which comprises means for securing this sleeve (7) against sliding and rotating, as well as a clamping device for retaining the entrenching screws, characterised thereby that this clamping device is formed by an eye (13), which is laterally arranged on the sleeve (7), with a conical opening (13a), a collet (14), which is seated in this opening and provided with an external thread (14a), for the entrenching screw (4, 5), and a nut (15) seated on the external thread for drawing the collet (14) into the conical opening (13a) and thus reducing the clear width of the collet (14) in order to thereby retain the entrenching screw, wherein the external thread (14a) of the collet has at least one flat (14b) and the corresponding section of the conical opening (13a) a constriction matched thereto in order to prevent rotation of the collet, that the portion of the collet compressible by the drawing into the conical opening has a spherical outer surface and the conical opening has two extension tongues (13d) which enable a pivoting of the collet (14) in a plane parallel to the sleeve axis (7), and that the extension tongues (13d) are provided with a toothing (13c) which is present on a cylindrically curved surface and meshes with the toothing (16a), which is provided at a washer (16) pushed onto the external thread (14a) of the collet (14) and retained there also by the nut (15).

## Revendications

1. Elément de fixation interne servant à relier rigidement une vertèbre de la colonne vertébrale à au moins une deuxième vertèbre de cette colonne vertébrale, constitué de deux vis de Schanz (4, 5) par vertèbre, de deux tiges filetées (6) et d'un élément de liaison pour chaque vis de Schanz afin de relier ces dernières de manière fixe à l'une des tiges filetées, chaque élément de liaison présentant une douille (7) pouvant coulisser et tourner sur ladite tige filetée (6) et comportant des moyens pour assurer ladite douille (7) contre tout déplacement et rotation, ainsi qu'un dispositif de serrage pour maintenir en place la vis de Schanz, caractérisé en ce que ce dispositif de serrage est formé d'un oeil (13) à ouverture conique (13a) disposé sur la douille (7), d'une pince de serrage (14) logée dans cette ouverture et munie d'un filetage extérieur (14a) pour la vis de Schanz (4, 5), et d'un écrou (15) monté sur le filet extérieur pour tirer la pince (14) dans l'ouverture conique (13a) et donc réduire l'ouverture de ladite pince (14) afin de bloquer ainsi la vis de Schanz, tandis que le filetage extérieur (14a) de ta pince de serrage présente au moins un méplat (14b) et que la section correspondante de l'ouverture conique (13a) est pourvue d'un rétrécissement adapté à ce méplat afin d'empêcher la rotation de la pince de serrage, en ce que la section de la pince de serrage compressible par son introduction dans l'ouverture conique présente une face extérieure de forme sphérique et en ce que l'ouverture conique de pattes de prolongement (13d), permette un basculement de la pince (14) dans un plan parallèle à l'axe de la douille (7), et en ce que les pattes de prolongement (13d) sont munies d'une denture (13c) pratiquée sur une face à courbure cylindrique, cette denture engrenant avec la denture (16a) dont est munie une rondelle d'appui (16) placée sur le filetage extérieur (14a) de la pince de serrage (14) et fixée à cet endroit par l'écrou (15).
